(19) Europäisches Patentamt
European Patent Office
Office européen des brevets

(11) **EP 4 751 732 A1**

(12) **EUROPEAN PATENT APPLICATION**
published in accordance with Art. 153(4) EPC

(43) Date of publication:
**03.06.2026 Bulletin 2026/23**

(21) Application number: **24845666.7**

(22) Date of filing: **25.07.2024**

(51) International Patent Classification (IPC):
**A61K 39/145** (2006.01)   **A61P 31/16** (2006.01)
**C12N 7/04** (2006.01)

(52) Cooperative Patent Classification (CPC):
**A61K 39/145; A61P 31/16; C12N 7/04**

(86) International application number:
**PCT/JP2024/026583**

(87) International publication number:
**WO 2025/023291 (30.01.2025 Gazette 2025/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **25.07.2023 JP 2023121102**

(71) Applicant: **KM Biologics Co., Ltd.
Kumamoto-shi, Kumamoto 860-8568 (JP)**

(72) Inventors:
• **OHYAMA, Yusuke
Kikuchi-shi, Kumamoto 869-1298 (JP)**
• **MATSUURA, Kenta
Kumamoto-shi, Kumamoto 860-8568 (JP)**
• **MIZUNOE, Shota
Kumamoto-shi, Kumamoto 860-8568 (JP)**

(74) Representative: **Graf von Stosch
Patentanwaltsgesellschaft mbH
Triftstraße 5
80538 München (DE)**

(54) **METHOD FOR PRODUCING INACTIVATED INFLUENZA VACCINE BY EGG CULTURE METHOD**

(57)   A method for producing an inactivated influenza vaccine by an egg culture method in accordance with the present disclosure includes a pathogen inactivation step of inactivating a pathogen that is contaminated in an egg and that is other than an influenza virus.

FIG. 1

```
VIRUS STRAIN FOR PRODUCTION
        │ INOCULATION
        ▼
CULTURE IN EMBRYONATED EGG
        │ COLLECT
        │ ALLANTOIC FLUID
        ▼
ULTRACENTRIFUGATION
        ▼
SUCROSE DENSITY GRADIENT
CENTRIFUGATION
        ▼
BPL INACTIVATION
        │ HYDROLYSIS
        ▼
ETHER TREATMENT
        ▼
ULTRAFILTRATION (UF)
        ▼
FORMALIN INACTIVATION
        ▼
SINGLE STOCK SOLUTION
```

EP 4 751 732 A1

**Description**

Technical Field

The present invention relates to a method for producing an inactivated influenza vaccine by an egg culture method.

Background Art

An influenza virus belongs to the Orthomyxoviridae family, which is classified as an "enveloped, negative-stranded, single-stranded RNA virus" in terms of virus classification, and type A, type B, type C, and type D are known. The difference in type is based on the difference in antigenicity between an M1 protein that is a structural protein and a nuclear protein (NP), among proteins constituting a virus particle. In addition to the above differences, there are also the pathological, morphological, and genetic differences, and the differences are large particularly between types A and B and types C and D. Types A and B are repeatedly prevalent every year and cause human influenza in many cases.

The influenza virus was isolated from a pig in 1931, and a human influenza virus was isolated in 1933. At the same time, the influenza virus was found to propagate in an embryonated egg. This has led to use of an inactivated whole-virus vaccine obtained by inactivating, with formaldehyde, a virus inoculated into an egg, cultured, harvested, and purified. A virus that is chemically modified with a viral gene or viral protein only loses infectivity. Thus, such a virus becomes a vaccine antigen while maintaining the particle shape.

However, the initial whole-virus inactivated vaccine had strong side effects such as a fever reaction, and a method for removing a lipid membrane component that is considered to cause such a reaction has been devised. For example, the following vaccine has high safety: a vaccine which contains, as a main component, an influenza antigen hemagglutinin (HA) serving as a prototype of the current split vaccine and in which a lipid membrane component that causes pyrexia has been removed with ether by separating the influenza antigen hemagglutinin (HA) with use of a surfactant after zonal ultracentrifugation purification. A split vaccine has been produced since 1964 up to the present (Non-patent Literature 1). It has been reported that, in a split vaccine, a nucleic acid of a virus and a high-molecular-weight protein are removed together with a splitting agent, and antigen components HA and neuraminidase (NA) are retained (Non-patent Literature 2).

A method in which an intended virus strain is inoculated into a hatching egg, propagated, and purified is widely carried out to produce an influenza vaccine. In Japan, the foregoing split vaccine containing purified HA (and NA) as a main antigen is in distribution. As an example of production of a seasonal influenza vaccine, type A and type B strains are each individually cultured in an embryonated egg as a seasonal strain, and an HA fraction is collected as a main antigen and formulated. HA is one of surface antigens of an influenza virus and is involved in adsorption of a virus to a host cell. Inoculation of this preparation produces an antibody against HA, and prevention of influenza is expected by the antibody acting as a protective antibody against the influenza virus.

A vaccine produced by propagating a virus in a cultured cell in place of a conventional egg is referred to as a cell culture influenza vaccine. Examples of a cell used include EB66 cells (derived from duck embryonic stem cells), MDCK cells (derived from canine renal tubular epithelial cells), and Vero cells (derived from African green monkey kidney epithelial cells). Although a cell culture method has advantages of flexibility and rapidity of vaccine production, an inactivated split vaccine produced by an egg culture method has become mainstream in a seasonal influenza vaccine in Japan. Regardless of a production method, a vaccine is inoculated into an animal such as a human and thus needs to have high safety. This requires inactivation of a propagated virus and purification of a main antigen of the vaccine with high purity. Furthermore, bio-derived raw materials are used in biopharmaceuticals such as a vaccine. Thus, in Japan, necessary measures that should be taken to ensure quality, efficacy, and safety of pharmaceuticals, etc. are established in "STANDARDS FOR BIOLOGICAL RAW MATERIALS" (Non-Patent Literature 3: MHLW Notification No. 37/Enacted on February 28, 2018). These bio-derived raw materials are bio-derived biopolymers, and thus have a risk of infection with a pathogen such as a virus. Also in a method for producing an influenza vaccine by the egg culture method, a step of inactivating or removing a pathogen caused by an egg which is a raw material is important.

In a typical method for producing an inactivated split influenza vaccine by the egg culture method, a total of four types of production strains, i.e., two type A strains and two type B strains of an influenza virus are each individually cultured in an embryonated egg. An allantoic fluid containing the propagated virus is harvested and subjected to steps, i.e., ultracentrifugation, sucrose density gradient centrifugation, ether treatment, and formalin inactivation, so that an undiluted vaccine (single stock solution) is prepared. After the single stock solution is prepared for the four types of production strains, the single stock solution is prepared by dilution with a buffer solution such that a specified amount of HA of each strain virus is contained, and is formulated.

In the typical method for producing an inactivated split influenza vaccine by the egg culture method, a step of inactivating the influenza virus is carried out as a final step of the production method by formalin treatment or the like. In a case where a pathogen is contaminated in an egg of a raw material, the pathogen has ordinarily been eliminated in a screening test on an embryonated egg or a subsequent purification step. However, since such elimination is not perfect, a stock solution from which a pathogen cannot be removed before preparation of the stock solution is discarded.

β-Propiolactone (BPL; $C_3H_4O_2$) is a β-lactone that has a molecular weight of 72.06 and that has ring strain, and is also written as 2-Oxetanone or Betaprone. BPL easily cleaves between O-$H_2$C and becomes an alkylating agent that attacks a nucleophilic functional group of a biopolymer, and thus is expected to bring about an effect of inactivating an influenza virus. Evaluation of BPL treatment with respect to an influenza virus is disclosed in, for example, Non-patent Literature 4.

Patent Literatures including Patent Literatures 1 and 2 disclose carrying out BPL treatment as a method for breaking down host cell nucleic acid associated with an influenza virus or a viral antigen thereof produced by cell culture.

Meanwhile, Patent Literature 3 considers introduction of a BPL treatment step regarding production of an inactivated influenza vaccine by the egg culture method. Patent Literature 3 indicates that carrying out formalin treatment with respect to an influenza virus treated with BPL in advance makes it possible to prevent reduction in innate immune activation capacity of an influenza vaccine antigen which reduction is caused by formalin. Patent Literature 3 also indicates that an object thereof is to provide: an inactivated influenza vaccine (specifically, a whole-virus vaccine) which involves formalin treatment and which has improved innate immune activation capacity (immunogenicity) of an influenza vaccine antigen; and a method for producing the inactivated influenza vaccine.

Citation List

[Patent Literature]

[Patent Literature 1]
Published Japanese Translation of PCT International Application, Tokuhyo, No. 2009-513694
[Patent Literature 2]
Published Japanese Translation of PCT International Application, Tokuhyo, No. 2012-507272
[Patent Literature 3]
International Publication No. WO 2021/172418

[Non-patent Literature]

[Non-patent Literature 1]
YAKUGAKU ZASSHI (Journal of the Pharmaceutical Society of Japan) 131 (12), 1723-1731 (2011): https://www.jstage.jst.go.jp/article/yakushi/131/12/131_ 12_17231 _pdf/-char/ja
[Non-patent Literature 2]
Frontiers in Immunology 2021, vol. 12, Article 711997
[Non-patent Literature 3]
"STANDARDS FOR BIOLOGICAL RAW MATERIALS" Enacted on February 28, 2018 (MHLW Notification No. 37)
[Non-patent Literature 4]
Vaccine 2019, vol. 37, pp. 1630-1637

Summary of Invention

Technical Problem

In a case where incompatibility of an undiluted influenza vaccine produced by the egg culture method occurs frequently due to contamination of a transovarially transmissible pathogen such as an avian reovirus (ARV), it is feared that the yield of undiluted multivalent (e.g., tetravalent) vaccines is unbalanced, so that product supply is impeded. Thus, measures against a transovarially transmissible pathogen are necessary in production of an influenza vaccine by the egg culture method. For example, regarding an avian reovirus, measures have been taken to administer a booster dose of inactivated Reo vaccines to breeding chickens. However, since these inactivated Reo vaccines are vaccines for minimizing the onset of symptoms, it is impossible to prevent the breeding chickens from being infected with ARV, and contamination of ARV in eggs occurs in a case where the breeding chickens are infected with ARV. Thus, realization of clearance of a transovarially transmissible pathogen in a method for producing an inactivated influenza vaccine by the egg culture method is required.

An aspect of the present invention is to establish, in a method for producing an inactivated influenza vaccine by an egg culture method, an inactivation step with respect to a pathogen that may be contaminated in an egg which is a raw material and that is other than an influenza virus.

Solution to Problem

The inventors of the present invention conducted diligent research in order to solve the above problems. As a result, the inventors of the present invention have found a step that, in a method for producing an inactivated influenza vaccine by an egg culture method, makes it possible to inactivate a pathogen which is contaminated in an egg. The inventors of the present invention accomplished the present invention by finding that quality including immunogenicity of an inactivated influenza vaccine produced by a production method including a step that makes it possible to inactivate a pathogen which is contaminated in an egg is unexpectedly unchanged.

A production method in accordance with an aspect of the present invention which production method has solved the above problems is a method for producing an inactivated influenza vaccine by an egg culture method, the method including a pathogen inactivation step of inactivating a pathogen that is contaminated in an egg and that is other than an influenza virus.

Advantageous Effects of Invention

According to an aspect of the present invention, a method for producing an inactivated influenza vaccine by an egg culture method makes it possible to inactivate a pathogen that may be contaminated in an egg which is a raw material and that is other than an influenza virus.

Brief Description of Drawings

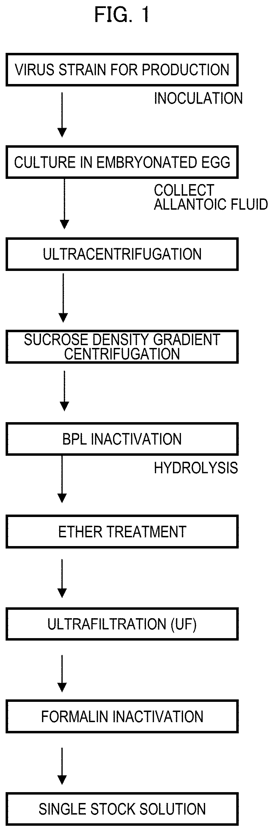

Fig. 1 shows a flowchart of a method for producing an inactivated influenza vaccine by an egg culture method in accordance with an aspect of the present invention.

Description of Embodiments

In the present specification, "A to B" means not less than A and not more than B unless otherwise specified.

[Method for producing inactivated influenza vaccine]

A method for producing an inactivated influenza vaccine by an egg culture method in accordance with an aspect of the present invention (hereinafter sometimes referred to as "the present production method") includes a pathogen inactivation step. Fig. 1 shows an example flow of the present production method.

(Inactivated influenza vaccine)

In the present specification, the inactivated influenza vaccine refers to a vaccine containing an inactivated influenza virus. Inactivation of the influenza virus will be described later.

(Influenza virus)

Examples of the influenza virus used in the present production method include type A, type B, type C, and type D, and subtypes of these. One type of influenza virus or two or more types of influenza viruses may be contained in the inactivated influenza vaccine. That is, the inactivated influenza vaccine produced by the present production method may be a monovalent vaccine or a multivalent vaccine.

The influenza virus used in the present production method may be a strain isolated from an infected animal or patient, or may be a recombinant virus strain genetically engineered and established in a cultured cell.

(Egg culture method)

In the present specification, the egg culture method refers to culturing an influenza virus strain by inoculating the influenza virus strain into an embryonated egg. For example, such culture need only be carried out such that an influenza virus strain is inoculated into an approximately 10-day-old embryonated egg and then cultured at 30°C to 37°C for 1 day to 7 days.

(Pathogen inactivation step)

In the pathogen inactivation step, a pathogen that is contaminated in an egg and that is other than an influenza virus is inactivated.

(Pathogen)

Examples of the pathogen that is contaminated in an egg include avian leukosis virus (ALV), egg drop syndrome 1976 virus (EDSV), avian reovirus (ARV), and Mycoplasma gallisepticum (Mg). In the present specification, ALV, EDSV, ARV, and Mg are sometimes collectively referred to as a "transovarially transmissible pathogen".

Examples of the pathogen inactivation step include a step of harvesting an allantoic fluid containing an influenza virus cultured by the egg culture method (hereinafter sometimes referred to as a "virus-containing allantoic fluid") and then inactivating a pathogen contained in the virus-containing allantoic fluid. Prior to inactivation of the pathogen contained in the virus-containing allantoic fluid, the virus-containing allantoic fluid may be subjected to centrifugation such as ultracentrifugation. Alternatively, prior to inactivation of the pathogen contained in the virus-containing allantoic fluid, the virus-containing allantoic fluid may be subjected to sucrose density gradient centrifugation or the like so as to be purified or concentrated.

(BPL inactivation step)

The pathogen inactivation step may be a step of treating the pathogen with β-propiolactone (hereinafter sometimes abbreviated to "BPL"). In the following step, such a step is sometimes referred to as a BPL inactivation step. In the BPL inactivation step, the pathogen can be inactivated, for example, by adding BPL to the virus-containing allantoic fluid.

In order to, for example, maintain efficiency of inactivation of the pathogen and quality of the influenza vaccine, the BPL added to the virus-containing allantoic fluid has a final concentration of preferably not less than 0.05 vol%, and more preferably not less than 0.08 vol%. Further, the final concentration of the BPL is preferably not more than 0.15 vol%, and more preferably not more than 0.1 vol%.

A treatment temperature at which a pathogen inactivation reaction is carried out in the BPL inactivation step may be room temperature (1°C to 30°C), or may be not lower than 3°C and not higher than 7°C. In order to, for example, maintain efficiency of inactivation of the pathogen and quality of the influenza vaccine, a treatment time for which the pathogen inactivation reaction is carried out in the BPL inactivation step is preferably not less than 1 minute, more preferably not less than 3 hours, even more preferably not less than 5 hours, and still more preferably not less than 10 hours. Further, a pathogen inactivation reaction time is preferably not more than 44 hours, and more preferably not more than 24 hours.

The pathogen inactivation reaction in the BPL inactivation step can be ended by heating the virus-containing allantoic fluid to 34°C to 40°C, then maintaining a heating state for 2 hours to 4 hours, and hydrolyzing the BPL.

Immunogenicity of the inactivated influenza vaccine produced by the present production method is unchanged by the pathogen inactivation reaction in the BPL inactivation step. In production of an influenza vaccine, it is usually feared that carrying out the inactivation step in two stages (pathogen inactivation and influenza virus inactivation) changes quality such as immunogenicity of the influenza vaccine. However, the inventors of the present invention have found that even carrying out the inactivation step in two stages does not change quality including immunogenicity of an inactivated influenza vaccine.

(Influenza virus inactivation step)

The present production method may further include, after the pathogen inactivation step, an influenza virus inactivation step of using formaldehyde to inactivate the influenza virus.

In the influenza virus inactivation step, the influenza virus can be inactivated, for example, by adding formalin to a treatment solution obtained after the pathogen inactivation step. In the present specification, the formalin refers to an aqueous solution containing 35% by mass to 41% by mass of formaldehyde.

In order to, for example, maintain efficiency of inactivation of the influenza virus and quality of the influenza vaccine, the formalin added to the virus-containing allantoic fluid has a final concentration of preferably not less than 0.003 vol%, and more preferably not less than 0.005 vol%. Further, the final concentration of the formalin is preferably not more than 0.03 vol%, and more preferably not more than 0.01 vol%.

An inactivation reaction of the influenza virus may be carried out at room temperature (1°C to 30°C), or may be carried out at not lower than 2°C and not higher than 6°C. In order to, for example, maintain efficiency of inactivation of the influenza virus and quality of the influenza vaccine, time of the inactivation reaction of the influenza virus is preferably not less than 7 days, and 14 days is a more preferable aspect. Further, the time of the inactivation reaction of the influenza virus is preferably not more than 21 days. Furthermore, it is also possible to select an inactivation treatment condition in which the inactivation reaction of the influenza virus is carried out at 20°C to 30°C for several days, for example, for 3 days.

(Delipidation step)

The present production method may further include, after the pathogen inactivation step and before the influenza virus inactivation step, a - step of treating the influenza virus with ether. The delipidation step makes it possible to produce an inactivated split vaccine.

In the delipidation step, for example, addition of ether to a treatment solution obtained after the pathogen inactivation step cleaves an influenza virus particle, so that a lipid component of the influenza virus particle can be removed. The treatment solution obtained after the pathogen inactivation step may be subjected to a filtration process such as microfiltration (MF) before the delipidation step.

Examples of the ether used in the delipidation step include diethyl ether and diisopropyl ether. One type of ether or two or more types of ethers may be used in the delipidation step.

The amount of the ether used in the delipidation step may be 10 vol% to 400 vol%, 12.5 vol% to 100 vol%, or 33 vol% to 50 vol%, relative to the total amount of the treatment solution obtained after the pathogen inactivation step.

In the delipidation step, a surfactant in addition to the ether may be used. Examples of the surfactant include polyoxyethylene octylphenyl ether, polysorbate 80, and combinations of these. The amount of the surfactant used in the delipidation step may be 0.002 vol% to 0.3 vol%, 0.005 vol% to 0.1 vol%, or 0.05 vol% to 0.075 vol%, relative to the total amount of the treatment solution obtained after the pathogen inactivation step.

A treatment temperature at which the delipidation step is carried out may be changed, as appropriate, in accordance with a type of the influenza virus, a type and a concentration of the ether used, and the like, and, for example, may be room temperature (1°C to 30°C), 4°C to 25°C, or 14°C to 24°C.

A treatment time for which the delipidation step is carried out may be changed, as appropriate, in accordance with a type of the influenza virus, a type and a concentration of the ether used, the treatment temperature, and the like, and, for example, may be 1 hour to 2 hours.

After the delipidation step, a delipidation reaction can be ended by removing the ether by centrifugation or the like.

[Influenza virus inactivated split vaccine or inactivated whole-virus vaccine]

An influenza virus inactivated split vaccine or inactivated whole-virus vaccine produced by the present production method (hereinafter sometimes referred to as "the present inactivated influenza vaccine") is encompassed in an aspect of the present invention.

Examples of a main antigen of the inactivated split vaccine include a hemagglutinin (HA) antigen and a neuraminidase (NA) antigen. The antigen of the inactivated split vaccine is preferably an HA antigen because the HA antigen has high immunogenicity.

The amount of the influenza virus contained in the present inactivated influenza vaccine may be 1 $\mu$g/ml to 40 $\mu$g/ml per virus strain as a hemagglutinin concentration.

The present inactivated influenza vaccine may include a carrier that is pharmaceutically acceptable. The carrier can be a carrier that is normally used in vaccine production. Specifically, examples of the carrier include saline, buffered saline, dextrose, water, glycerol, an isotonic aqueous buffer solution, and combinations of these. To the vaccine, an emulsifier, a preservative (e.g., thimerosal), a tonicity agent, a pH adjusting agent, an inactivator (e.g., formalin), and/or the like may be further added, as appropriate.

In order to further enhance immunogenicity, the present inactivated influenza vaccine may further contain an adjuvant. Examples of the adjuvant include: an aluminum adjuvant or an oil-in-water squalene-containing emulsion adjuvant (AS03, MF59, etc.); toll-like receptor ligands such as CpG and 3-O-deacylated-4'-monophosphoryl lipid A (MPL); a saponin-based adjuvant and a polymer-based adjuvant such as poly-$\gamma$-glutamic acid; and polysaccharides such as chitosan and inulin.

A dosage form of the present inactivated influenza vaccine may be, for example, a liquid, a powder (freeze-dried powder, dried powder), a capsule, a tablet, or a frozen state.

An administration route of the present inactivated influenza vaccine may be, for example, dermal administration, sublingual administration, ophthalmic administration, intradermal administration, intramuscular administration, oral administration, enteral administration, nasal administration, intravenous administration, subcutaneous administration, intraperitoneal administration, and oral pulmonary inhalation. A method for administering the present inactivated influenza vaccine may be, for example, a method of administration with use of a syringe, a percutaneous patch, a microneedle, an implantable sustained-release device, a microneedle-equipped syringe, a needle-free device, or a spray.

Examples of a target to which the present inactivated influenza vaccine is to be administered include humans and nonhuman animals, and more specific examples of the target include vertebrate animals such as birds and mammals. Examples of the mammals include laboratory animals such as mice, rats, rabbits, guinea pigs, rhesus monkeys, crab-eating macaques, and nonhuman primates; pet animals (pets) such as dogs and cats; domestic animals such as pigs, cattle, goats, sheep, and horses; and humans.

The dose of the present inactivated influenza vaccine and the number of times the present inactivated influenza vaccine is administered can be selected, as appropriate, in accordance with a symptom degree, age, gender, body weight, and/or an administration form.

Aspects of the present invention can also be expressed as follows:

A production method in accordance with Aspect 1 of the present invention is a method for producing an inactivated influenza vaccine by an egg culture method, the method including a pathogen inactivation step of inactivating a pathogen that is contaminated in an egg and that is other than an influenza virus.

In Aspect 2 of the present invention, a method may be configured such that, in Aspect 1 of the present invention, the pathogen is at least one type of pathogen selected from the group consisting of avian leukosis virus (ALV), egg drop syndrome 1976 virus (EDSV), avian reovirus (ARV), and Mycoplasma gallisepticum (Mg).

In Aspect 3 of the present invention, a method may be configured such that, in Aspect 1 or 2 of the present invention, the pathogen inactivation step is a step of treating the pathogen with β-propiolactone.

In Aspect 4 of the present invention, a method may be configured such that, in Aspect 3 of the present invention, the β-propiolactone has a final concentration of not less than 0.05 vol% and not more than 0.15 vol% in the pathogen inactivation step.

In Aspect 5 of the present invention, a method may be configured such that, in Aspect 3 or 4 of the present invention, treatment with the β-propiolactone in the pathogen inactivation step is carried out at a temperature of not lower than 3°C and not higher than 7°C for a time of not less than 1 minute and not more than 24 hours.

In Aspect 6 of the present invention, a method may be configured such that, in any one of Aspects 3 to 5 of the present invention, immunogenicity of the inactivated influenza vaccine is unchanged by treatment with the β-propiolactone.

An influenza virus inactivated split vaccine or inactivated whole-virus vaccine in accordance with Aspect 7 of the present invention is produced by a method of any one of Aspects 1 to 6.

In Aspect 8 of the present invention, a method may be configured, in any one of Aspects 1 to 6, to further include, after the pathogen inactivation step, an influenza virus inactivation step of using formaldehyde to inactivate the influenza virus.

In Aspect 9 of the present invention, a method may be configured, in Aspect 8, to further include, after the pathogen inactivation step and before the influenza virus inactivation step, a delipidation step of treating the influenza virus with ether.

An influenza virus inactivated split vaccine or inactivated whole-virus vaccine in accordance with Aspect 10 of the present invention is produced by a method in accordance with Aspect 8.

An influenza virus inactivated split vaccine in accordance with Aspect 11 of the present invention is produced by a method in accordance with Aspect 9.

The following description will more specifically discuss an embodiment of the present invention with reference to Examples. It is a matter of course that the present invention is not limited to the Examples below and that details of the present invention can have various aspects. Further, the present invention is not limited to the embodiments, but can be altered by a skilled person in the art within the scope of the claims. An embodiment derived from a proper combination of technical means disclosed in respective different embodiments is also encompassed in the technical scope of the present invention. Furthermore, all the documents listed in the present specification are incorporated herein by reference.

Examples

[Example 1] Preparation of inactivated influenza vaccine (with pathogen inactivation step)

An inactivated influenza vaccine was prepared in accordance with a flow of a production method of Fig. 1. Influenza virus B/Brisbane/60/2008 strain was inoculated into the allantoic cavity of an 11-day-old embryonated egg, and an allantoic fluid containing the influenza virus was harvested after culture at 34°C for 2 days to 3 days. The influenza virus collected by subjecting the harvested allantoic fluid to concentration and ultracentrifugation was allowed to resuspend in a phosphate-buffered sodium chloride solution (PBS, pH 7.4) and subjected to sucrose density gradient centrifugation. By collecting a fraction containing the influenza virus, purification was carried out. BPL was added to the purified virus solution so as to achieve a final concentration of 0.1 vol%, and a pathogen inactivation reaction was carried out at 5°C for 22 hours. Thereafter, the pathogen inactivation reaction was ended by heating at 37°C for 2 hours.

A delipidation treatment was carried out by adding diethyl ether (100 vol%) to a treatment solution obtained after the pathogen inactivation reaction, and stirring a resulting solution at room temperature for 1 hour. After the delipidation treatment, the diethyl ether was removed by centrifugation to collect a virus suspension solution (hemagglutinin (HA) fraction). The collected virus suspension solution was subjected to ultrafiltration to remove a BPL decomposition product. An aqueous formaldehyde-containing solution was added so as to achieve a formalin final concentration of 0.005 vol%. Then, after an influenza virus inactivation reaction was carried out under refrigeration for 14 days, a desired inactivated influenza vaccine was obtained.

[Reference Example 1] Preparation of inactivated influenza vaccine (without pathogen inactivation step)

An inactivated influenza vaccine was prepared in accordance with a procedure similar to that in Example 1, except that a pathogen inactivation reaction with use of BPL and ultrafiltration (removal of a BPL decomposition product) after a delipidation treatment were not carried out.

An antigen of the inactivated influenza vaccine prepared in each of Example 1 and Reference Example 1 is an HA antigen.

[Evaluation Example 1] Clearance test of pathogen contaminated in egg

(1) Test substance

The allantoic fluid obtained after purification by sucrose density gradient centrifugation in Example 1 was used as a test substance in Evaluation Example 1.

(2) Sample

One volume of spike virus was added to 9 volumes of the test substance in a glass beaker, and a resulting mixture was stirred with a stirrer at room temperature for 1 minute and dichotomized into polypropylene (PP) conical tubes. To one of these, a BPL stock solution was added at a final concentration of 0.05 vol% or 0.1 vol%. Then, a resulting solution subjected to gentle inversion mixing 10 times at room temperature was transferred to a new PP conical tube and then subdivided in equal amounts into four PP cryogenic vials by sampling time points (0 hour, 5 hours, 10 hours, and 19 hours). The subdivided solutions were contaminated with use of a rotator in a cool and dark place (5°C) until the respective sampling time points. After sampling, the solutions including the vials were rapidly transferred into a constant temperature water tank at 37°C and reacted at 37°C for 2 hours to decompose BPL.

(3) Results of clearance test of pathogen contaminated in egg

In light of employment of an egg culture method in production of an influenza vaccine, among pathogens that may be derived from chickens, a total of five types, which are not only four types, i.e., avian leukosis virus (ALV), egg drop syndrome 1976 virus (EDSV), avian reovirus (ARV), and Mycoplasma gallisepticum (Mg), but also Pseudorabies virus (PRV) that is widely used in a clearance test of a virus, etc. were selected as examples of an infectious pathogen (Table 1). Among these five types, transovarially transmissible nonenveloped viruses are ARV (an RNA virus) and EDSV (a DNA virus).

[Table 1]

Table 1

| Model pathogen | Family | Envelope | Nucleic acid |
|---|---|---|---|
| ALV | Retrovirus | Present | RNA |
| EDSV | Adenovirus | Absent | DNA |
| ARV | Reovirus | Absent | RNA |
| Mg | Mycoplasma | - | - |
| PRV | Herpes virus | Present | DNA |

Pathogens listed in Table 1 were individually added (spiked) to the test substance to calculate the degree of reduction of each pathogen before and after a BPL treatment step by the following Equation (1), and inactivation ability of the BPL treatment step with respect to the pathogen was evaluated. In the following description, the pathogens listed in Table 1 are each sometimes referred to as a "virus, etc.".

LRV = log (titer of virus, etc. in sample before BPL treatment step/titer of virus, etc. in sample after BPL treatment step)     (1)

In Equation (1), LRV refer to a logarithmic reduction value.

Four kinds of BPL treatment time, which are 0 hour, 5 hours, 10 hours, and 19 hours, were set for each of two conditions, i.e., BPL final concentrations of 0.1 vol% and 0.05 vol%, and the inactivation ability of the BPL treatment step with respect to the pathogen was evaluated twice for each of the concentrations. The BPL treatment time being 0 indicates that sampling was carried out immediately after inversion mixing was carried out 10 times with BPL added, and corresponds to an operation in which measurement time is less than 1 minute.

Table 2 shows evaluation results. Note here that the WHO shows an understanding that LRV being 4 or more falls under a robust, highly reliable, and effective step in a viral clearance test (WHO Technical Report, Series No. 924, 2004, Annex 4). However, due to a viral titer and a limit of quantification in the sample before the BPL treatment step, LRV is sometimes less than 4 even if the virus, etc. is cleared.

[Table 2]

Table 2

| Pathogen | BPL final concentration | | 0 hour | 5 hours | 10 hours | 19 hours |
|---|---|---|---|---|---|---|
| ALV | 0.05 vol% | First time | >3.9 | >3.9 | >3.9 | >3.9 |
| | | Second time | >3.9 | >3.9 | >3.9 | >3.9 |
| | 0.1 vol% | First time | >3.7 | >3.7 | >3.7 | >3.7 |
| | | Second time | >3.9 | >3.9 | >3.9 | >3.9 |
| EDSV | 0.05 vol% | First time | 3.7 | 3.9 | >4.8 | >4.9 |
| | | Second time | 3.8 | >4.1 | >4.9 | >4.8 |
| | 0.1 vol% | First time | >4.9 | >4.9 | >4.9 | >4.9 |
| | | Second time | >5.0 | >5.0 | >5.0 | >5.0 |
| ARV | 0.05 vol% | First time | >3.8 | >4.0 | >4.1 | >4.1 |
| | | Second time | >4.4 | >4.4 | >4.4 | >4.4 |
| | 0.1 vol% | First time | >4.2 | >4.2 | >4.2 | >4.2 |
| | | Second time | >4.1 | >4.1 | >4.1 | >4.1 |
| Mg | 0.05 vol% | First time | ≥8.03 | ≥8.03 | ≥8.03 | ≥8.03 |
| | | Second time | ≥8.14 | ≥8.14 | ≥8.14 | ≥8.14 |
| | 0.1 vol% | First time | ≥8.10 | ≥8.10 | ≥8.10 | ≥8.10 |
| | | Second time | ≥8.12 | ≥8.12 | ≥8.12 | ≥8.12 |
| PRV | 0.05 vol% | First time | >4.7 | >4.7 | >4.7 | >4.7 |
| | | Second time | >4.7 | >4.7 | >4.7 | >4.7 |

(continued)

| Pathogen | BPL final concentration | | 0 hour | 5 hours | 10 hours | 19 hours |
|---|---|---|---|---|---|---|
| | 0.1 vol% | First time | >4.9 | >4.9 | >4.9 | >4.9 |
| | | Second time | >4.6 | >4.6 | >4.6 | >4.6 |
| ALV: Avian leukosis virus<br>EDSV: Egg drop syndrome 1976 virus<br>ARV: Avian reovirus<br>Mg: Mycoplasma gallisepticum<br>PRV: Pseudorabies virus | | | | | | |

As shown in Table 2, in a case where the BPL final concentration was 0.1 vol%, for EDSV, ARV, Mg, and PRV, LRV was 4 or more, and no pathogen was detected, regardless of the treatment time (0 hour, 5 hours, 10 hours, and 19 hours) at 5°C. For ALV, the result of ">3.7" (first time) or ">3.9" (second time) was obtained due to the viral titer and the limit of quantification before the BPL treatment step. However, it can be said that the virus, etc. was cleared also for ALV. That is, in a case where the BPL final concentration was 0.1 vol%, none of spike viruses, etc. including ARV were detected, and clearance was confirmed for all types of pathogens, regardless of the treatment time (0 hour, 5 hours, 10 hours, and 19 hours) at 5°C.

In a case where the BPL final concentration was 0.05 vol%, no spike virus, etc. was detected for ALV, PRV and Mg, regardless of the treatment time. In contrast, for EDSV, a surviving virus was detected in samples sampled at 0 hour and 5 hours after the BPL treatment, but clearance was confirmed at 10 hours and 19 hours. In contrast, for ARV, a surviving virus was detected in a sample sampled at 0 hour after the BPL treatment, but clearance was confirmed at 5 hours, 10 hours, and 19 hours.

[Evaluation Example 2] Mouse immunogenicity (HI antibody titer)

To a group of 10 female mice (3 weeks old, ddY strain), 0.5 mL of each inactivated influenza vaccine prepared in Example 1 (with the pathogen inactivation step) and 0.5 mL of each inactivated influenza vaccine prepared in Reference Example 1 (without the pathogen inactivation step) were intraperitoneally administered once so as to achieve approximately 0.6 $\mu$gHA (equivalent value) per mouse. Blood was collected after immunization for 21 days, and an HI antibody titer in a serum was evaluated. Table 3 shows a geometric mean and a 95% confidence interval of the HI antibody titer. The HI antibody titer with and without the 0.1 vol% BPL treatment step was evaluated for three batches of each of four types of influenza virus strains.

A result of carrying out F-test in Table 3 has shown that all strains other than the Victoria lineage had equal variance. Thus, Student's T-test (two-tailed test with a significance level of 5%) was carried out. The result of the F-test has shown that the Victoria lineage had unequal variance. Thus, Welch's T-test (two-tailed test with a significance level of 5%) was carried out. The results of the tests have shown that no significant difference in the HI antibody titer depending on the presence or absence of the BPL treatment step was found in all the strains, and no influence on mouse immunogenicity due to carrying out the BPL treatment step was detected.

[Table 3]

Table 3

| | With pathogen inactivation step | | Without pathogen inactivation step | |
|---|---|---|---|---|
| | Batch number | HI antibody titer[a] | Batch number | HI antibody titer[a] |
| A/Brisbane/02/2018 (IVR190) (H1N1) pdm09 | A/B-a01 | 279 (177-439) | A/B-b01 | 121 (68-217) |
| | A/B-a02 | 137 (41-462) | A/B-b02 | 139 (60-321) |
| | A/B-a03 | 211 (97-462) | A/B-b03 | 260 (120-565) |
| A/Kansas/14/2017 (X-327) (H3N2) | A/K-a01 | 279 (59-1309) | A/K-b01 | 160 (44-588) |
| | A/K-a02 | 106 (44-255) | A/K-b02 | 520 (268-1009) |
| | A/K-a03 | 160 (63-408) | A/K-b03 | 260 (140-483) |

(continued)

| | With pathogen inactivation step | | Without pathogen inactivation step | |
|---|---|---|---|---|
| | Batch number | HI antibody titer[a] | Batch number | HI antibody titer[a] |
| B/Phuket/3073/2013 (Yamagata lineage) | B/P-a01 | 113 (58-221) | B/P-b01 | 260 (154-440) |
| | B/P-a02 | 171 (105-281) | B/P-b02 | 184 (110-307) |
| | B/P-a03 | 149 (79-283) | B/P-b03 | 160 (83-310) |
| B/Maryland/15/2016 (NYMCBX-69A) (Victoria lineage) | B/M-a01 | 113 (45-282) | B/M-b01 | 98 (62-158) |
| | B/M-a02 | 86 (38-196) | B/M-b02 | 46 (20-106) |
| | B/M-a03 | 32 (18-58) | B/M-b03 | 86 (33-221) |
| **[a] A range in parentheses indicates a 95% confidence interval.** | | | | |

[Evaluation Example 3] Hemagglutination activity (HA titer)

Chicken erythrocytes were used to measure an HA titer of each of the inactivated influenza vaccines prepared in Example 1 and Reference Example 1. Protein concentrations in strain stock solutions were equally prepared, and the strain stock solutions were applied to a 96-well V-bottom microplate. Chicken erythrocytes and a clear hemagglutination reaction were observed in all the strains. Furthermore, no difference in reactivity with chicken erythrocytes was found between the stock solutions depending on the presence or absence of the BPL treatment step.

As described above, addition of the pathogen inactivation step with the BPL treatment revealed no difference in HI antibody titer or HA titer, and it has been confirmed that active pharmaceutical ingredients have equivalent and homogeneous qualities in the presence or absence of the BPL treatment step.

[Evaluation Example 4] Comparison of frequency of occurrence of incompatibility with adventitious virus testing in production of inactivated influenza vaccine due to presence or absence of BPL treatment step

(1) Adventitious virus testing (primary cultured chicken kidney cell inoculation test)

Adventitious virus testing was carried out with reference to "Adventitious virus testing method" (Minimum Requirements for Veterinary Biological Products: Amended on June 30, 2020 (Notification No. 1246)), which is a method for examining the absence of an adventitious virus that can be detected in a sample of a live vaccine and a serum. A case where no CPE was found in a cultured cell during the observation period was considered to be compatible with this testing.

(2) Results before and after introduction of BPL treatment step

The frequency of occurrence of incompatibility of the inactivated influenza vaccines prepared in Reference Example 1 with the adventitious virus testing was approximately 4% on average. In contrast, no incompatibility of the inactivated influenza vaccine prepared in Example 1 with the adventitious virus testing was found.

Industrial Applicability

The production method of the present invention makes it possible to provide an inactivated influenza vaccine that has improved clearance of a transovarially transmissible pathogen, and such an inactivated influenza vaccine can be used particularly in the medical field.

**Claims**

1. A method for producing an inactivated influenza vaccine by an egg culture method, the method comprising a pathogen inactivation step of inactivating a pathogen that is contaminated in an egg and that is other than an influenza virus.

2. The method as set forth in claim 1, wherein the pathogen is at least one type of pathogen selected from the group consisting of avian leukosis virus (ALV), egg drop syndrome 1976 virus (EDSV), avian reovirus (ARV), and Mycoplasma gallisepticum (Mg).

3. The method as set forth in claim 1, wherein the pathogen inactivation step is a step of treating the pathogen with β-propiolactone.

4. The method as set forth in claim 3, wherein the β-propiolactone has a final concentration of not less than 0.05 vol% and not more than 0.15 vol% in the pathogen inactivation step.

5. The method as set forth in claim 4, wherein treatment with the β-propiolactone in the pathogen inactivation step is carried out at a temperature of not lower than 3°C and not higher than 7°C for a time of not less than 1 minute and not more than 24 hours.

6. The method as set forth in claim 3, wherein immunogenicity of the inactivated influenza vaccine is unchanged by treatment with the β-propiolactone.

7. An influenza virus inactivated split vaccine or inactivated whole-virus vaccine produced by a method recited in any one of claims 1 to 6.

8. The method as set forth in any one of claims 1 to 6, further comprising, after the pathogen inactivation step, an influenza virus inactivation step of using formaldehyde to inactivate the influenza virus.

9. The method as set forth in claim 8, further comprising, after the pathogen inactivation step and before the influenza virus inactivation step, a delipidation step of treating the influenza virus with ether.

10. An influenza virus inactivated split vaccine or inactivated whole-virus vaccine produced by a method recited in claim 8.

11. An influenza virus inactivated split vaccine produced by a method recited in claim 9.

## FIG. 1

```
┌─────────────────────────────────┐
│   VIRUS STRAIN FOR PRODUCTION   │
└─────────────────────────────────┘
              │  INOCULATION
              ▼
┌─────────────────────────────────┐
│    CULTURE IN EMBRYONATED EGG   │
└─────────────────────────────────┘
              │  COLLECT
              │  ALLANTOIC FLUID
              ▼
┌─────────────────────────────────┐
│       ULTRACENTRIFUGATION       │
└─────────────────────────────────┘
              │
              ▼
┌─────────────────────────────────┐
│   SUCROSE DENSITY GRADIENT      │
│       CENTRIFUGATION            │
└─────────────────────────────────┘
              │
              ▼
┌─────────────────────────────────┐
│        BPL INACTIVATION         │
└─────────────────────────────────┘
              │  HYDROLYSIS
              ▼
┌─────────────────────────────────┐
│        ETHER TREATMENT          │
└─────────────────────────────────┘
              │
              ▼
┌─────────────────────────────────┐
│      ULTRAFILTRATION (UF)       │
└─────────────────────────────────┘
              │
              ▼
┌─────────────────────────────────┐
│      FORMALIN INACTIVATION      │
└─────────────────────────────────┘
              │
              ▼
┌─────────────────────────────────┐
│      SINGLE STOCK SOLUTION      │
└─────────────────────────────────┘
```

## INTERNATIONAL SEARCH REPORT

| International application No. |
| --- |
| **PCT/JP2024/026583** |

| A. | CLASSIFICATION OF SUBJECT MATTER |
| --- | --- |

*A61K 39/145*(2006.01)i; *A61P 31/16*(2006.01)i; *C12N 7/04*(2006.01)i
FI:  A61K39/145; A61P31/16; C12N7/04

According to International Patent Classification (IPC) or to both national classification and IPC

| B. | FIELDS SEARCHED |
| --- | --- |

Minimum documentation searched (classification system followed by classification symbols)

A61K39/145; A61P31/16; C12N7/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2010-104365 A (DENKA SEIKEN CO., LTD.) 13 May 2010 (2010-05-13) | 1, 2, 7 |
| | claims, paragraphs [0005], [0006], example 3 | |
| Y | | 1-11 |
| X | WO 2022/210763 A1 (DENKA CO., LTD.) 06 October 2022 (2022-10-06) | 7 |
| | claim 1, examples 1, 2 | |
| Y | | 1-11 |
| X | WILSON, David S. A. et al. Evaluation of Mycoplasma Inactivation during Production of Biologics: Egg-Based Viral Vaccines as a Model. Applied and Environmental Microbiology. 2010, vol. 76, no. 9, pp. 2718-2728 | 1-8, 10, 11 |
| | abstract, etc. | |
| Y | | 1-11 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **17 September 2024** | **08 October 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT

International application No.

**PCT/JP2024/026583**

### C.    DOCUMENTS CONSIDERED TO BE RELEVANT

| Category* | Citation of document, with indication, where appropriate, of the relevant passages | Relevant to claim No. |
|---|---|---|
| X | PAWAR, S. D. et al. Evaluation of different inactivation methods for high and low pathogenic avian influenza viruses in egg-fluids for antigen preparation. Journal of Virological Methods. 2015, vol. 222, pp. 28-33<br>    abstract, etc. | 7 |
| Y | | 1-11 |
| A | JP 2009-513694 A (NOVARTIS VACCINES AND DIAGNOSTICS GMBH & CO. KG) 02 April 2009 (2009-04-02)<br>    claims | 1-11 |
| A | WO 2014/039978 A2 (SZATHMARY, DR. Susan) 13 March 2014 (2014-03-13)<br>    claims | 1-11 |

**INTERNATIONAL SEARCH REPORT**
Information on patent family members

International application No.

**PCT/JP2024/026583**

| Patent document cited in search report | | | Publication date (day/month/year) | Patent family member(s) | | | Publication date (day/month/year) |
|---|---|---|---|---|---|---|---|
| JP | 2010-104365 | A | 13 May 2010 | US | 2011/0182940 | A1 | |
| | | | | claims, paragraphs [0005], [0006], example 3 | | | |
| | | | | WO | 2010/038719 | A1 | |
| | | | | EP | 2351835 | A1 | |
| | | | | CN | 102171334 | A | |
| WO | 2022/210763 | A1 | 06 October 2022 | US | 2024/0156943 | A1 | |
| | | | | claim 1, examples 1, 2 | | | |
| | | | | EP | 4316515 | A1 | |
| | | | | CN | 117098552 | A | |
| | | | | KR | 10-2023-0157397 | A | |
| | | | | AU | 2022251654 | A | |
| JP | 2009-513694 | A | 02 April 2009 | WO | 2007/052163 | A2 | |
| | | | | claims | | | |
| | | | | US | 2009/0304729 | A1 | |
| | | | | EP | 1951296 | A2 | |
| | | | | KR | 10-2008-0081254 | A | |
| | | | | CN | 101365480 | A | |
| WO | 2014/039978 | A2 | 13 March 2014 | US | 2016/0082094 | A1 | |
| | | | | EP | 2943219 | A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009513694 W
- JP 2012507272 W
- WO 2021172418 A

### Non-patent literature cited in the description

- **YAKUGAKU ZASSHI**. *Journal of the Pharmaceutical Society of Japan*, 2011, vol. 131 (12), 1723-1731, https://www.jstage.jst.go.jp/article/yakushi/131/12/131_ 12_17231 _pdf/-char/ja
- *Frontiers in Immunology*, 2021, vol. 2 (711997)
- *STANDARDS FOR BIOLOGICAL RAW MATERIALS*, 28 February 2018 (37)
- *Vaccine*, 2019, vol. 37, 1630-1637
- Minimum Requirements for Veterinary Biological Products: Amended. *Adventitious virus testing method*, 30 June 2020 (1246)